Europäisches Patentamt

European Patent Office

Office européen des brevets

(11) Veröffentlichungsnummer: **0 318 663**
**A1**

(12) **EUROPÄISCHE PATENTANMELDUNG**

(21) Anmeldenummer: **88116082.4**

(22) Anmeldetag: **29.09.88**

(51) Int. Cl.⁴: **C12N 15/00 , C12P 13/08 , C12N 1/20 , //(C12N1/20, C12R1:15),(C12N1/20, C12R1:19)**

(30) Priorität: **06.11.87 DE 3737729**

(43) Veröffentlichungstag der Anmeldung:
**07.06.89 Patentblatt 89/23**

(84) Benannte Vertragsstaaten:
**BE CH DE FR GB LI NL SE**

(71) Anmelder: **Degussa Aktiengesellschaft**
**Weissfrauenstrasse 9**
**D-6000 Frankfurt am Main 1(DE)**

Anmelder: **Kernforschungsanlage Jülich**
**GmbH**
**Wilhelm-Johnen-Strasse**
**D-5170 Jülich(DE)**

(72) Erfinder: **Sahm, Hermann, Prof.**
**Wendelinusstrasse 71**
**D-5170 Jülich(DE)**
Erfinder: **Eggeling, Lothar, Dr.**
**Schneppruthweg 7**
**D-5170 Jülich(DE)**
Erfinder: **Menkel, Elke, Dr.**
**Goerdelerstrasse 34**
**D-6100 Darmstadt(DE)**
Erfinder: **Thierbach, Georg, Dr.**
**Am Meierteich 11**
**D-4800 Bielefeld(DE)**
Erfinder: **Aragon, Julio, Dr.**
**Am Vorwerk 25**
**D-4800 Bielefeld(DE)**

(54) **Pendelvektor pZ1, rekombinantes, ein Aspartasegen enthaltendes Plasmid, seine Verwendung zur Herstellung von Aminosäuren der Aspartat-Familie aus Fumarat-haltigem Nährmedium und es enthaltende Mikroorganismen.**

(57) Die Erfindung betrifft einen Pendelvektor, der durch Fusion der Plasmide pHM 1519 und pACYC 177 entsteht, ein in Corynebacterium glutamicum replizierbares rekombinantes Plasmid, zusammengesetzt aus dem Pendelvektor und einem chromosomalem DNA-Fragment, das ein Aspartase-Gen trägt, die Verwendung dieses rekombinanten Plasmids in einem Bakterium der Gattung Corynebacterium glutamicum zur Herstellung von Aminosäuren der Aspartat-Familie in einem Fumarat-haltigen Medium und dieses Plasmid enthaltenden Mikroorganismen.

Abb. 1
Restriktionskarte des Plasmids pZ1. Der fett gezeichnete Teil stellt den pHM1519- und der dünn gezeichnete Teil den pACYC177-Teil dar.

## Pendelvektor pZ1, rekombinantes, ein Aspartasegen enthaltendes Plasmid, sein Verwendung zur Herstellung von Aminosäuren der Aspartat-Familie aus Fumarat-haltigem Medium und es enthaltende Mikroorganismen

Die Erfindung betrifft den Pendelvektor pZ1, der durch Fusion der Plasmide pHM 1519 und pACYC 177 entsteht, ein in Corynebacterium glutamicum replizierbares rekombinantes Plasmid, zusammengesetzt aus dem Pendelvektor und einem chromosomalen DNA-Fragment, das ein Aspartase-Gen trägt, und die Verwendung dieses rekombinanten Plasmids in einem Bakterium der Gattug Corynebacterium glutamicum zur Herstellung von Aminosäuren in einem Fumarat-haltigen Medium.

Die Aminosäuren Lysin, Threonin, Isoleucin etc., die aus Asparat gebildet werden, gehören zu den essentiellen Aminosäuren und sind daher von besonderer Bedeutung. Sie finden weite Verwendung vor allem als Nahrungs- und Tierfutterzusatz, als Bestandteil von Infusionslösungen sowie als Reagenzien in der pharmazeutischen und chemischen Industrie.

In den letzten dreißig Jahren sind neben enzymatischen Syntheseverfahren vor allem mikrobielle Verfahren zur Produktion von Lysin entwickelt worden. Dabei werden hauptsächlich Mikroorganismen der Gattung Corynebacterium und Brevibacterium eingesetzt, die große Mengen an Aminosäuren ins Fermentationsmedium ausscheiden, nachdem die Regulationsmechanismen der Biosynthesewege durch Mutationen ausgeschaltet wurden.

Ein anderer Weg zur Beeinflussung der Aminosäureproduktion durch Mikroorganismen besteht in der Anwendung genetischer Rekombinationstechniken.

So haben Untersuchungen ergeben, daß die Bildung von Lysin von der Menge an zur Verfügung stehendem Aspartat abhängt (Shiio, I et al. J. Biochem. 84 (1978) 647-657), so daß es vorteilhaft zu sein scheint, für die Produktion dieser Aminosäure eine möglichst große Menge ans Aspartat bereitzustellen.

Einen Ansatz bietet die Klonierung des Phosphoenolpyruvatcarboxylase-Gens aus Brevibacterium in Brevibacterium lactofermentum, wie es in der EP-A1-0143 195 (Sano et al) beschrieben wird.

Gegenstand der vorliegenden Erfindung ist die Erhöhung des Aspartatpools durch Klonierung des Aspartase-Gens aus E.-coli in Corynebacterium glutamicum.

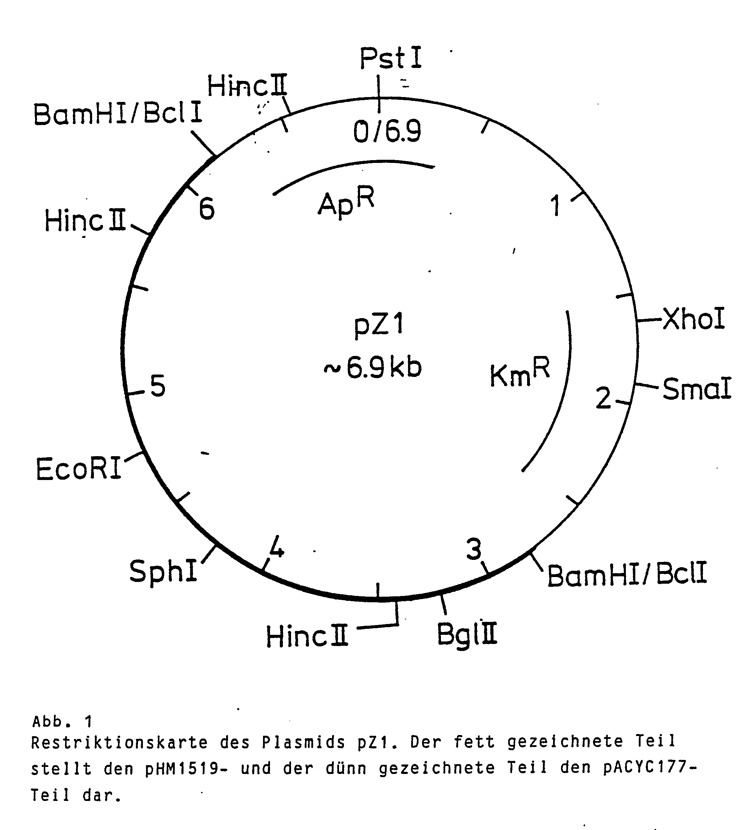

Die Voraussetzung dafür bildet ein in Corynebactrium glutamicum replizierbarer Pendelvektor pZ1 mit einer Größe von 6,9 kb, erhalten durch Fusion des kryptischen Plasmids pHM 1519 aus Corynebacterium glutamicum 13058 und dem E.-coli Plasmid pACYC 177, der die in der in Figur 1 abgebildeten Restriktionskarte enthaltenen Schnittstellen aufweist.

Die Plasmide und Verfahren zu ihrer Isolierung sind aus der Literatur bekannt ( pHM 1519: Miwa et al., Agric. Biol. Chem. 48 (1984) 2901-2903; pACYC: Chang et al., J. Bact. 134 (1978) 1141-1157).

Zur Konstruktion des erfindungsgemäßen Pendelvektors wird das Plasmid pHM 1519 (3,0 kb) nach an sich bekannten Metho den mit dem Restriktionsenzym Bcl I geschnitten, das E.-coli-Plasmid pACYC 177 (3,8 kB) mit dem Enzym BamH I.

Die dabei entstehenden sticky ends sind komplementär, was eine einfache Fusion der Plasmide ermöglicht. Nach der Ligation bildet sich ein Fusionsplasmid, genannt pZ 1, von~6,9 kB, das in Corynebacterium glutamicum und in Escherichia coli replizierte (Abb.1). Das Plasmid pZ 1 trägt die Antibiotikaresistenzen für Ampicillin und Kanamycin, die als Selektionsmarker in Escherichia coli genutzt werden können; in Corynebacterium führt nur die Kanamycinresistenz zu einem funktionsfähigen Protein. Dieses so konstruierte rekombinante DNA-Mplekül besitzt verschiedene Restriktionsschnittstellen, die für Klonierungen in Escherichia coli und Corynebacterium glutamicum benutzt werden können.

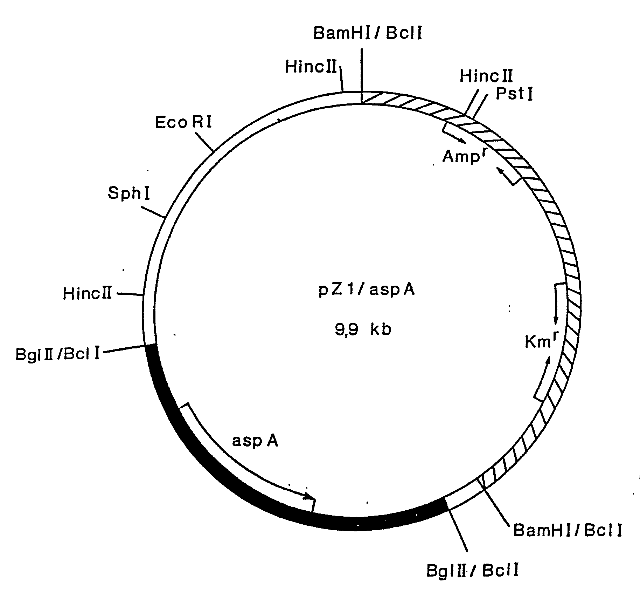

Weiterer Bestandteil der Erfindung ist ein in Corynebacterium glutamicum replizierbares rekombinantes Plasmid, das dadurch gekennzeichnet ist, daß es ein chromosomales DNA-Fragment enthält, das ein von einem Aspartase bildenden Bakterium der Gattung E.-coli abgeleitetes Aspartase-Gen trägt, eine Größe von ca 9,9 kB hat, und die in der in Figur 2 abgebildeten Restriktionskarte enthaltenen Spaltstellen aufweist.

Aspartase (EC 4.3.1.1), ein Enzym, das in der Gattug Corynebacterium nicht vorhanden ist, katalysiert die Aminierung von Fumarsäure mit Ammonium zu Asparat. Mit Hilfe dieser Reaktion kann man in einem Reaktionsschritt Asparat für die Aminosäuresynthese aus Fumarsäure nachliefern. Das Gen, das in Escherichia coli für die Aspartase kodiert, wurde von Guest et al. (J. of Gen.Micorbiol. 130 (1984) 1271-1278) kloniert und kann nach dem dort beschriebenen Verfahren in bekannter Weise z.B. mit Hilfe des E.-coli-Vektors pBR 322 leicht in E.-coli K 12 exprimiert werden.

Mit Hilfe des Restriktionsenzyms Bcl I wird das Aspartase-Gen als 2,8 kB-Fragment aus einem Escherichia coli-Vektor pBR 322 herausgeschnitten und in die einzige Bgl II-Schnittstelle des Vektors pZ 1 kloniert. Die Transformation des rekombinanten DNA-Moleküls in die Recipienten erfolgt beispielsweise über Protoplasten nach einem von Santamaria modifizierten Verfahren. (Santamaria et al. J.ofBact. 162 (1985) 463-467, J. of General Microbiology 130 (1984) 2237-2246.

Als Recipienten der rekombinanten DNA werden bevorzugt mutierte Stämme von Corynebacterium glutamicum verwendet, die bereits bestimmte Aminosäuren bilden und ausscheiden. Repräsentative Beispiele solcher Recipienten sind als Lysinproduzenten homoserinauxotrophe, leucinauxotrophe und S-(2-aminoethyl)cystein-resistente Stämme von Corynebacterium glutamicum oder Brevibacterium flavum.

Mikroorganismen, die das chromosomale Spender-DNA-Fragment enthalten und damit das gewünschte Aspartase-Gen, können aus den wie vorstehen beschrieben hergestellten Transformanden leicht aufgrund der im Pendelvektor pZ 1 lokalisierten Kanamycin-Resistenz ausselektiert werden

Das aus Escherichia coli stammende Aspartase-Gen wird in den Stämme der Gattung Corynebacterium mit hoher Aktivität exprimiert, was durch Enzymmessungen nach dem von Williams und Lartigue beschriebenen Verfahren nachgewiesen wurde (Methods in Enzymology 13 (1969), 354-355).

Die Erfindung betrifft auch die Verwendung des Plasmids in den entsprechenden Transformante zur Herstellung von Lysin, Threonin oder Isoleucin in einem ein Alkalifumarat, bevorzugt Natriumfumarat, enthaltenden Nährmedium.

Die zu verwendenden Medien zur Inkubation der Transformante sind synthetische, halbsynthetische oder natürliche Medien, wie sie häufig zur Inkubation von Bakterien verwendet werden. Sie enthalten Nährstoffe, wie Kohlenstoffquellen, Stickstoffquellen und anorganische Verbindungen. Beispiele günstiger Kohlenstoffquellen sind Zucker, wie Glucose Fructose, Invertzucker, verzuckerte Stärke, Sorbit oder Glycerin. Beispiele günstiger Stickstoffquellen sind Ammoniumsulfat, Ammoniumchlorid, Ammoniumnitrat, Ammoniumphosphat, Ammoniumhydroxid, Ammoniumtartrat, Ammoniumacetat oder Harnstoff. Beispiele günstiger Nährstoffe, die sowohl als Stickstoff, als auch als Kohlenstoff-Quelle angeboten werden können, sind Pepton Fleischextrakt oder Maisquellwasser. Beispiele günstiger anorganischer Verbindungen sind Kaliumdihydrogenphosphat, Dikaliumhydrogenphosphat, Natriumhydrogenphosphat. Dinatriumhydrogenphosphat, Magnesiumsulfat, Magnesiumchlorid, Kaliumchlorid, Natriumchlorid, Eisen(II)-sulfat, Eisen(II)-chlorid, Eisen-(III)-sulfat, Eisen(III)-chlorid, Mangansulfat oder Manganchlorid. Beispiele weiterer günstiger Nährstoffe sind Hefeextrakt oder Vitamine.

Zudem weist das Nährmedium bevorzugt eine Biotinkonzentration von mehr als 50 μg/l auf, da sonst das Wachstum suboptimal ist. Weiterhin setzt man der Fermentationsbrühe vorzugsweise Kanamycin in einer Konzentration von 50 μg/ml zu, den Selektionsdruck auf die plasmidtragenden Bakterien aufrecht zuerhalten. Die Fermentation wird unter aeroben Bedingungen, bei einem pH-Wert von 5-9 und 25 bis 40° C 48 bis 144 Stunden durchgeführt, wobei Lysin in der Nährlösung akkumuliert.

Um die vorteilhaften Eingenschaften der transformierten Mikroorganismen zu nutzen, ist es notwendig, eine Natriumfumaratkonzentration von 60 bis 125 mM im Fermentationsmedium vorzugeben. Die Substanz kann aber auch portionsweise mit fortschreitender Umsetzung zugesetzt werden.

Die Isolierung und Reinigung der Aminosäure aus der Nährlösung erfolgt mittels bekannter Methoden.

Erfindungsgemäß eingesetzt als Recipienten der rekombinanten DNA werden bevorzugt Stämme von Corynebacterium glutamicum ATCC 13032, die bereits Mutationen zur Herstellung von Lysin tragen.

Die verwendeten Stämme DG 52-5 und MH 20-22B können leicht aus den Stämmen DG 52-5/pZ 1 aspA (DSM 4208)und MH 20-22 BpZ 1 aspA (DSM 4207) erhalten werden, indem man das erfindungsgemäße Plasmid pZ 1 entfernt, ohne die Gastgeberzelle zu schädigen. Die genannten Stämme wurden bei der DSM nach dem Budapester Vertrag hinterlegt.

Methoden für derartige Operationen sind beschrieben in Bact. Rev. 36 (1972) 361-504 und J.Bacteriol. 88 - (1964) 261.

Es zeigt sich, daß die transformierten Stämme über eine verbesserte Fähigkeit verfügen, Lysin in einem Fumarat-haltigen Nährmedium zu bilden.

In der folgenden Tabelle sind die Ergebnisse der in den Beispeilen aufgeführten Versuche zusammengefaßt.

Beispiel 1

Isolierung von Plasmid pACYC177

Plasmid pACYC177 ist ein Plasmid mit einer Größe von 3,8 Kb Länge; es vermittelt in Escherichia coli Resistenz gegen Ampicillin und Kanamycin (Chang,A.C.Y. and Cohen, S.N., Journal of Bacteriology 134, 1141-1156 (1978)).

Plasmid pACYC177 wurde folgendermaßen isoliert: Zellen des Stammes Escherichia coli ATCC37031 wurden bei 30°C bis zur stationären Wachstumsphase in einem Liter Stl-Medium kultiviert, welches folgende Zusammensetzung hat: 7,8 g/l Pepton aus Fleisch; 5,6 g/l NaCl und 5,0 g/l Glucose. Die durch Zentrifugation vom Kulturmedium abgetrennten Zellen wurden in 50 ml eines Puffers von pH 8, bestehend aus 103 g/l Saccharose; 25 mM Tris; 25 mM EDTA und 40.000 U/ml Lysozym, suspendiert und 10 Minuten bei Raumtemperatur inkubiert. Die Zellsuspension wurde durch Zugabe von 25 ml einer Lösung, bestehend aus 0,3 M NaOH und 20 g/l Natriumdodecylsulfat, lysiert. Das Lysat wurde mit 20 ml einer mit demineralisiertem Wasser gesättigten Phenollösung extrahiert und anschließend die wäßrige Phase von der Phenol-haltigen Phase durch Zentrifugation getrennt. Der wäßrigen Überstand (70 ml) wurde nach Zugabe von 0,1 Volumenteil 3 M Natriumacetat durch Zugabe von 1,1 Volumenteilen Isopropanol bei Raumtemperatur gefällt. Das Präzipitat wurde in 5 ml TE-Puffer von pH 8, bestehend aus 10 mM Tris; 1 mM EDTA und 100 µg/ml Ribonuklease, gelöst und 1 Stunde bei 37°C inkubiert. Nach Extraktion mit oben beschriebener Phenollösung und Trennung der Phenol-haltigen von der wäßrigen Phase durch Zentrifugation wurde die DNA-haltige wäßrige Phase, durch Zugabe von Isopropanol - wie oben beschrieben - gefällt. Das Sediment wurden in 1 ml des oben beschriebenen TE-Puffers gelöst und enthielt ungefähr 200 µg pACYC177-DNA.

Beispiel 2

## Isolierung von Plasmid pHM1519

Plasmid pHM 1519 ist ein Plasmid mit einer Größe von 3,0 Kb Länge, welches in Corynebacterium glutamicum repliziert (Miwa, K. et al, Agricultural and Biological Chemistry 48, 2901-2903 (1984)).

Plasmid pHM1519 wurde folgendermaßen isoliert: Zellen des Stammes Corynebacterium glutamicum ATCC13058 wurden bei 30°C bis zur stationären Wachstumsphase in einem Liter StlP-Medium kultiviert, welches folgende Zusammensetzung hat: 7,8 g/l Pepton aus Fleisch; 7,8 g/l Pepton aus Casein: 2,8 g/l Hefeextrakt; 5,6 g/l NaCl; 5,0 g/l Glucose und 30 U/l Penicillin G. Die durch Zentrifugation vom Kulturmedium abgetrennten Zellen wurden in 50 ml eines Puffers von pH 8, bestehend aus 103 g/l Saccharose; 25 mM Tris; 25 mM EDTA und 160.000 U/ml Lysozym, suspendiert und 1 Stunde bei 37°C inkubiert. Die Zellsuspension wurde durch Zugabe von 25 ml einer Lösung, bestehend aus 0,3 m NaOH und 20 g/l Natriumdodecylsulfat, lysiert. Das Lysat wurde mit 20 ml einer mit demineralisiertem Wasser gesättigten Phenollösung extrahiert und anschließend die wäßrige Phase von der Phenol-haltigen Phase durch Zentrifugation getrennt. Der wäßrige Überstand (70 ml) wurde nach Zugabe von 0,1 Volumenteil 5 M Natriumacetat durch Zugabe 1, 1 Volumenteilen Isopropanol bei Raumtemperatur gefällt. Das Präzipitat wurde in 5 ml TE-Puffer von pH 8, bestehend aus 10 mM Tris; 1 mM EDTA und 100 µg/ml Ribonuklease, gelöst und 1 Stunde bei 37°C inkubiert. Nach Extraktion mit oben beschriebener Phenollösung und Trennung der Phenol-haltigen Phase von der wäßrigen Phase durch Zentrifugation wurde die DNA-haltige wäßrige Phase durch Zugabe von Isopropanol-wie oben beschrieben - gefällt. Das Sediment wurde in 1 ml des oben beschriebenen TE-Puffers gelöst und enthielt ungefähr 150 µg oben beschriebenen TE-Puffers gelöst und enthielt ungefähr 150 µg pHM1519-DNA.

Beispiel 3

## Konstruktion eines Pendelvektors

Plasmid pACYC177-DNA (0,5 µg) wurde mit 2 Einheiten des Restriktionsenzyms BamHI (bezogen von der Firma BRL) bei 37° und einer Inkubationszeit von 1 Stunde linearisiert und anschließend mit 1 Einheit alkalischer Phosphatase (bezogyen von der Firma Boehringer) dephosporyliert.

Plasmid pHM1519-DNA(1,0 µg) wurde mit 4 Einheiten des Restriktionsenzyms BclI (bezogen von der Firma BRL) bei 50°C und einer Inkubationszeit von 1 Stunde linearisiert.

Die oben beschriebenen linearisierten Plasmid-DNAs wurden gemischt und nach 5-minütigem Erhitzen bei 65°C mit Hilfe von 1U T4-DNA-Ligase in Gegenwart von ATP und Mercaptoethanol bei 12°C und einer Inkubationszeit von 14 Stunden ligiert. Der Ligationsansatz mit den in ihm enthaltenen Pendelvektoren wurde - wie unten beschrieben - zur Transformation eingesetzt.

Kompetente Zellen von Escherichia coli DH5 (bezogen von der Firma BRL) wurden nach Angabe des

4

Herstellers mit oben beschriebenem Ligationsansatz transformiert. Transformanten wurden durch Bebrütung t 37° C) auf Stl-Aar (siehe 1.) in Gegenwar von Kanamycin (25 µg/ml) und Ampicillin (50 µg/ml) selektioniert. Von den Transformanten wurde der Stamm DM272-3 ausgewählt und für weitere Untersuchungen verwendet.

Die DNA des Pendelvektors, der die Bezeichnung pZ1 erhielt, wurde aus Stamm DM272-3 - wie in 1. beschrieben - isoliert. Die Plasmid-DNA wurde mit verschiedenen Restriktionsenzymen verdaut und anschließend einer Agarosegel-Elektrophorese unterzogen. (Maniatis, T. et al; Molecular Cloning, A Laboratory Manual: Cold Spring Harbor Laboratory, 1982.) Durch Vergleich mit Standard-DNA-Fragmenten wurde die Länge des Pendelvektors pZ1 mit 6,9 Kb bestimmt. Die Restriktionskarte des Plasmides ist in Abb. 1 dargestellt.

Beispiel 4

Replikation des Pendelvektors pZ1 in Corynebacterium glutamicum

Es wird gezeigt, daß der Pendelvektor pZ1 in Corynebacterium glutamicum repliziert, und daß das Kanamycinresistenz-Gen von pZ1, welches von pACYC177 stammt, in Corynebacterium glutamicum exprimiert wird. Als Empfängerstamm wurde Corynebacterium glutamicum ATCC13032 benutzt.

Protoplasten von Corynebacterium glutamicum ATCC13032 wurden in Anlehnung an Santamaria et al. (Journal of General Microbiology 130, 2237-2246 (1984)) folgendermaßen hergestellt.

Der oben bezeichnete Stamm wurde bei 30° C in 25 ml MMYE-Medium (Katsumata. R. et al., Journal of Bacteriology 159, 306-311 (1984)) bis zur frühen exponentiellen Wachstumsphase kultiviert. Dann wurde Penicillin G (Endkonzentration 0,3 U/ml) hinzugegeben und die Kultur 3 Stunden weitergeführt. Die Zellen wurden durch Zentrifugation geerntet und anschließend in 5 ml eines Puffers, bestehend aus 0,41 M Saccharose; 0,01 MN $MgCl_2$; 50 % MMYE-Medium und 13,000 U/ml Lysozym, 12 Stunden bei 33° C bebrütet. Die Protoplastensuspension wurde durch 10-minütige Zentrifugation bei 1.000 g sedimentiert mit TSMC-Puffer von pH 7,5, bestehend aus 500 mM $Na_2$-Succinat 500 mM, 10 mM $MgCl_2$, 20 mM $CaCl_2$ und 50 mM Tris, gewaschen und in 1 ml TSMC-Puffer resuspendiert.

100 µl der Protoplastensuspension wurden mit ungefähr 2,4 µg pZ1 DNA, die - wie in 3. beschrieben - hergestellt worden war, vermischt und anschließend 700 µl einer Lösung, bestehend aus 7,5 ml TSMC-Puffer und 2,5 g Polyethylenglykol , hinzugegeben und 3 Minuten bei. Raumtemperatur inkubiert. Die Suspension wurde anschließend zu 7,2 ml TSMC-Puffer gegeben, der mit 1 g/l Hefeextrakt; 1 g/l Casamino Acids und 1 g/l Glucose supplementiert worden war. Nach einer Inkubationszeit von 3 Stunden wurden die transformierten Protoplasten durch 10-minütige Zentrifugation bei 1.000 g sedimentiert und in 1 ml TSMC-Puffer resuspendiert.

Diese Suspension wurde auf dem von Katsumata et al (Journal of Bacteriology 159, 306-311 (1984)) beschrieben RCG-AGar ausplattiert, der mit 300 µg/ml Kanamycin supplementiert worden war. Nach einer Bebrütung von 8 Tagen bei 30° C wurden 7 Kolonien, von denen eine als Stamm DM274-2 bezeichnet wurde, weiter untersucht. Alle 7 untersuchten Stämme waren in der Lage, auf Stl-/AGar, der mit 300 µg/ml Kanamycin supplementiert worden war, zu wachsen. Plasmid-DNA wurde aus den Stämmen - wie in 2. beschrieben - isoliert und - wie in 3. beschrieben - untersucht. Die Plasmid-DNA hatte die gleiche Größe und Restriktionskarte wie pZ1.

Den Pendelvektor pZ1 enthaltende Stämme von Corynebacterium glutamicum beziehungsweise Escherichia coli wurden unter den Nummern DSM 4241 beziehungsweise DSM 4242 bei der Deutschen Sammlung für Mikroorganismen (DSM) nach dem Budapester Vertrag hinterlegt.

Beispiel 5

In einem 500 ml Erlenmeyerkolben mit 2 Schikanen wurden 100 ml einer Nährlösung mit folgender Zusammensetzung gegeben:

| | |
|---|---|
| 40 g/l | Glucose x $H_2O$ |
| 20 g/l | $(NH_4)_2SO_4$ |
| 0,5 g/l | $K_2HPO_4$ |
| 0,5 g/l | $KH_2PO_4$ |
| 0,25 g/l | $MgSO_4$ x 7 $H_2O$ |
| 0,01 g/l | $FeSO_4$ x 7 $H_2O$ |
| 0,01 g/l | $MnSO_4$ x 4 $H_2O$ |
| 20 g/l | $CaCO_3$ |

Glucose wurde getrennt sterilisiert, $CaCO_3$ trocken sterilisiert (8 Stunden bei 150° C) und beides der Nährlösung steril zugesetzt.

Die Fermentation wurde mit einer 16 Stunden alten Vorkultur von Corynebacterium glutamicum DG 52-5 $(AEC^R)$ angeimpft, so daß die Anfangszelldichte einer Optischen Dichte bei 600 nm von 0,5 bis 0,8 entsprach. Die Hauptkultur wurde bei 30° C und 130 rpm inkubiert.

Nach einer Inkubationszeit von 72 Studnen enthielt das Fermentationsmedium 20 mM Lysin.

Beispiel 6

Es wurde, wie in Beispiel 5 beschrieben verfahren, jedoch enthielt das Nährmedium 125 mM Natriumfumarat. Nach einer Kultivierungsdauer von 72 Stunden enthielt das Fermentationsmedium 30 mM Lysin.

Beispiel 7

Es wurde, wie in Beispiel 6 beschrieben, verfahren, jedoch wurde für die Fermentation der Stamm mit einkloniertem Aspartasegen eingesetzt - Corynebacterium glutamicum DG 52-5/pZ1 aspA. Die Analyse des Fermentationsmediums ergab eine Lysinproduktion von 37 mM nach 72 h Kultivierung.

Beispiel 8

Bei diesen Fermentationsversuchen wurde ein anderer Lysinproduzent eingesetzt, der AEC-resistent und Leucinauxotroph ist - Corynebacterium glutamicum MH-20-22B. Das Fermentationsmedium enthielt dieselben Komponenten, wie in Beispiel 5 beschrieben, zusätzlich wurden 100 mg/l Leucin zugesetzt. Nach einer Inkubationszeit von 72 h enthielt das fermentationsmedium 30 mM Lysin.

Beispiel 9

Es wurde, wie in Beispiel 8 beschrieben, verfahren, jedoch enthielt das Nährmedium zusätzlich 60 mM Natriumfumarat. Bei diesem Versuchsansatz waren nach 72 h 55 mM lysin gebildet worden.

Beispiel 10

Es wurde, wie in Beispiel 9 beschrieben, verfahren, jedoch wurde der Stamm Corynebacterium glutamicum MH 20-22B/pZ1 aspA (aspA-Kürzel für Aspartasegen) eingesetzt. Nach einer Inkubationszeit von 72 h enthielt das Fermentationsmedium 71 mM Lysin.

In der Tabelle 1 sind die Ergebnisse noch einmal zusammengefaßt.

Tabelle 1

| Stamm | Fumarat eingesetzt (mM) | Lysin gebildet (mM) |
|---|---|---|
| C. glutamicum | | |
| 5. DG-52-5 | 0 | 20 |
| 6. DG-52-5 | 125 | 30 |
| 7. DG-52-5/pZ1 aspA (DSM 4208) | 125 | 37 |
| C. glutamicum | | |
| 8. MH 20-22B | 0 | 30 |
| 9. MH 20-22B | 50 - 125 | 55 |
| 10. MH 20-22B/pZ1 aspA (DSM 4207) | 50 - 125 | 71 |

**Ansprüche**

1. Pendelvektor pZ1 mit einer Größe von~6,9 kb,erhalten durch Fusion des kryptischen Plasmids pHM 1519 aus Corynebacterium glutamicum 13058 und dem E.-coli Plasmid pACYC 177, der die in der in Figur 1 abgebildeten Restriktionskarte enthaltenen Schnittstellen aufweist, wobei Figur 1 Bestandteil dieses Anspruchs ist.

2. In Corynebacterium glutamicum replizierbares rekombinates Plasmid gemäß Anspruch 1, dadurch gekennzeichnet, daß es zusätzlich ein chromosomales DNA-Fragment enthält, das ein von einem Aspartase bildenden Bakterium der Gattung E.-coli abgeleitetes Aspartase-Gen trägt, eine Größe von ca. 9,9 kb hat, und die in der in Figur 2 abgebildeten Restriktionskarte enthaltenen Schnittstellen aufweist, wobei Figur 2 Bestandteil dieses Anspruchs ist.

3. Verwendung des Plasmids gemäß Anspruch 2, als Vektor zur Transformation eines Aminosäuren der Aspartat-Familie produzierenden Mikroorganismus der Gattung Corynebacterium glutamicum.

4. Verwendung des Plasmids gemäß Anspruch 2 in einem, damit transformierten Stamm der Gattung Corynebacterium glutamicum zur Herstellung von Lysin in einem Fumarat-haltigen Nährmedium.

5. Lysin-produzierender transformierter Mikroorganismus DSM 4207.

6. Lysin-produzierender transformierter Mikroorganismus DSM 4208.

Abb. 1
Restriktionskarte des Plasmids pZ1. Der fett gezeichnete Teil stellt den pHM1519- und der dünn gezeichnete Teil den pACYC177- Teil dar.

BamHI / BclI
HincII
EcoRI
HincII
PstI
SphI
Amp^r
HincII
pZ1/aspA
9,9 kb
BglII/BclI
Km^r
aspA
BamHI/BclI
BglII/BclI

Abb. 2

## EINSCHLÄGIGE DOKUMENTE

EP 88116082.4

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP - A1 - 0 093 611 (AJINOMOTO CO., INC.)<br><br>* Ansprüche 1,3,5,6 *<br><br>-- | 1 | C 12 N 15/00<br>C 12 P 13/08<br>C 12 N 1/20<br>//(C 12 N 1/20<br>C 12 R 1:15)<br>(C 12 N 1/20<br>C 12 R 1:19) |
| A | EP - A2 - 0 158 940 (AJINOMOTO CO., INC.)<br><br>* Zusammenfassung *<br><br>-- | 1,3 | |
| A | EP - A2 - 0 219 027 (KYOWA HAKKO KOGYO CO., LTD.)<br><br>* Zusammenfassung *<br><br>-- | 1,3,4 | |
| A | EP - A1 - 0 197 335 (KYOWA HAKKO KOGYO CO., LTD.)<br><br>* Zusammenfassung *<br><br>-- | 1,3,4 | RECHERCHIERTE SACHGEBIETE (Int. Cl.4) |
| D,A | EP - A1 - 0 143 195 (AJINOMOTO CO., INC.)<br><br>* Ansprüche *<br><br>-- | 1,3,4 | C 12 N<br>C 12 P |
| A | EP - A2 - 0 158 201 (HOECHST AKTIENGESELLSCHAFT)<br><br>* Zusammenfassung *<br><br>---- | 1 | |

Der vorliegende Recherchenbericht wurde für alle Patentansprüche erstellt.

| Recherchenort | Abschlußdatum der Recherche | Prüfer |
|---|---|---|
| WIEN | 21-12-1988 | WOLF |